# EUROPEAN PATENT APPLICATION

(11) **EP 2 808 022 A1**
(43) Date of publication of application: **03.12.2014**
(21) Application number: 14174022.5
(22) Date of filing: 08.08.2011
(51) Int. Cl.: A61K 31/4439, A61K 9/20, A61K 9/50, A61K 9/16

(54) **Pharmaceutical compositions of metabotropic glutamate 5 receptor (MGLU5) antagonists**

(30) Priority: 11.08.2010 US 372693 P
(62) Divisional of application: 11746215.0
(71) Applicant: F. Hoffmann-La Roche AG, 4070 Basel (CH)
(72) Inventor: Chatterji, Ashish, East Brunswick, NJ New Jersey 08816 (US); Huang, Jingjun, Monmouth Junction, NJ New Jersey 08852 (US); Buurman, Stephanie, 4125 Riehen (CH); Lindenstruth, Kai, 10557 Berlin (DE); Sandhu, Harpreet K., West Orange, NJ New Jersey 07052 (US); Shah, Navnit Hargovindas, Clifton, NJ New Jersey 07012 (US)
(74) Representative: Poppe, Regina

(57) **Abstract**

Pharmaceutical compositions of metabotropic glutamate 5 receptor (mGlu5) antagonists or a pharmacologically acceptable salt thereof are disclosed. The compositions contain the therapeutic active compound with non-ionic polymer and ionic polymer, binder and fillers in matrix tablet. The compositions provide a pH-independent *in vitro* release profile with NMT 70 % in one hour, NMT 85 % in 4 hour, and NLT 80 % in 8 hours. The compositions are useful for the treatment of CNS disorders, such as Treatment-Resistant Depression (TRD) and Fragile X Syndrome.

## Description

The present invention provides a multi-particulate composition which comprises a compound of formula I 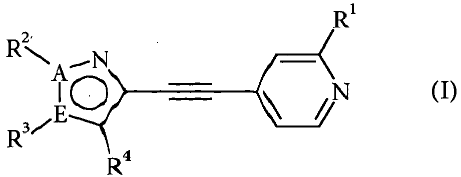 wherein
one of A or E is N and the other is C;
- R¹: is halogen or cyano;
- R²: is lower alkyl;
- R³: is aryl or heteroaryl, each of which is optionally substituted by one, two or three substituents chosen from halogen, lower alkyl, lower alkoxy, cycloalkyl, lower haloalkyl, lower haloalkoxy, cyano, or NR'R",
or by
1-morpholinyl,
1-pyrrolidinyl, optionally substituted by (CH₂)ₘOR,
piperidinyl, optionally substituted by (CH₂)ₘOR,
1,1-dioxo-thiomorpholinyl, or
piperazinyl, optionally substituted by lower alkyl or (CH₂)ₘ-cycloalkyl;
- R: is hydrogen, lower alkyl or (CH₂)ₘ-cycloalkyl;
- R' and R": are each independently hydrogen, lower alkyl, (CH₂)ₘ-cycloalkyl or (CH₂)ₙOR;
- m: is 0 or 1;
- n: is 1 or 2; and
- R⁴: is CHF₂, CF₃, C(O)H, or CH₂R⁵, wherein R⁵ is hydrogen, OH, C₁-C₆-alkyl or C₃-C₁₂-cycloalkyl;
and pharmaceutically acceptable salts thereof, a rate-controlling polymer, and a pH responding polymer.

The composition comprises the form of a matrix tablet, matrix pellets, or layered pellets.

The mGlu5 antagonists can exist in an amorphous form, a solvate or form a solid dispersion, co-crystal, or complex with other ingredients.

Many chemical entities are poorly water soluble and possess a pH-dependent solubility. This poor solubility raises significant hurdles in developing a reproducible drug PK profile with minimum food effect, which in turn affects the *in vivo* efficacy and safety of the drug.

There are several technical difficulties in the development of poorly soluble, weakly basic compounds. These difficulties include dose dumping due to high solubility of the compound in gastric fluid. Poor solubility and inadequate dissolution rate in the intestine result in lower absorption and bioavailability. Poor solubility also results in high inter and intra subject variability in the pharmacokinetics requiring a wider safety margin. Further, the effect of food on bioavailability and PK profiles complicates the dosing regimen.

Several modified release technologies are known, such as matrix tablet, pellet, osmotic pump, etc. These technologies were developed mainly for controlled delivery of water-soluble compounds. However, they often prove inadequate for poorly soluble or practically insoluble drugs because of their low solubility and variability in their release in the GI tract.

The emergence of newer therapeutic agents and a growing understanding of both pharmacokinetics and the physiological needs of patients make the task of controlled drug delivery more complex. For example, for poorly water soluble, weakly basic compounds with highly pH dependent solubility, there has been very limited success in providing adequate enhancement of a reproducible drug plasma profile within the therapeutic window. The limited success observed with these approaches was mainly related to a highly pH dependent solubility profile and an extremely low solubility in physiological intestinal fluids. The success of controlled delivery of this type of compound depends on improvement of drug release rate in intestine fluid, a pH-independent release profile in both gastric and intestinal fluid, and minimum inter and intra variation in drug release/absorption among subjects.

Several drug delivery technologies have been developed to address these concerns. Each of these technologies has certain detriments to the development of a drug composition having a pH independent dissolution.

One such method applies a delayed release enteric polymer coating to reduce dose dumping. Generally, this approach applies a thick layer of enteric polymer to delay the release of drug until reaching the intestinal tract. The high solubility of the drug in the low pH gastric fluid provides the strong driving force for drug dissolution and diffusion. However, this approach results in local irritation, fast absorption, high Cmax, and CNS side effects. The problem associated with this technology is an unpredictable PK profile due to inter and intra variation in gastric transit time and food effect.

Another composition strategy to provide pH independent drug release of a weakly basic drug in the GI tract is to incorporate organic acids as microenviromental pH modifiers. For example, the pH-independent release of fenoldopam from pellets with insoluble film coats has been shown. However, these compositions present several issues, such as salt conversion, control of diffusion of small molecular weight acidic pH modifiers, and potential interaction of organic acids with membranes that result in sigmoidal release profiles.

Due to their poor solubility in intestinal fluid, the absorption/bioavailability of some compounds is dissolution rate limited. Reduction in particle size can improve the dissolution rate, which can provide better absorption potential and likely improved therapeutics. Wet milling and nano-technology are two techniques that can be applied to poorly water-soluble drugs. Formation of a salt, co-crystal, solid dispersion, solvate, or amorphous form increases kinetic solubility of the compound, which provides a higher concentration gradient for drug release.

Size reduction and drug form modification are technologies that only reduce inter and intra variation and food effect to a limited extent. pH will still have a big impact on the solubility and dissolution rate of the compound, especially for poorly water soluble, basic compounds.

Controlling drug release by combining polymers has been demonstrated in the literature; however, these systems are designed to provide a zero order release profile. Furthermore, release rate is sensitive to the pH-dependency of the drug's solubility. Such systems do not have a means to increase the dissolution rate at higher pH.

The present invention provides a layered pellet composition which comprises an inert core, a layer comprising a compound of formula I or a salt thereof as defined herein, and a controlled release layer comprising a rate controlling polymer.

The present invention also provides methods for the formation of such compositions. The compositions are useful for the treatment of CNS related disorders, including treatment resistant depression (TRD) and Fragile-X syndrome.

To reproducibly control drug release *in vivo,* a soluble polymer, such as polyvinyl alcohol, polyvinyl pyrrolidone, or hypermellose and pH independent insoluble polymer, such as ethylcellulose, polyvinylacetate, or polymethacrylate can be applied in a coating or a matrix. pH is the driving force for dissolution of poorly water-soluble, basic compounds through a membrane or gel layer. The dissolution rate and absorption rate of such compounds are affected by variation in physiological pH of GI tract. Thus, pH will still have a big impact on the solubility of such drugs.

### Brief Description of the Drawings

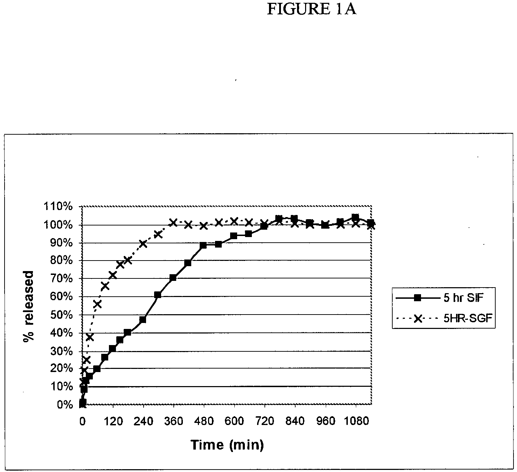

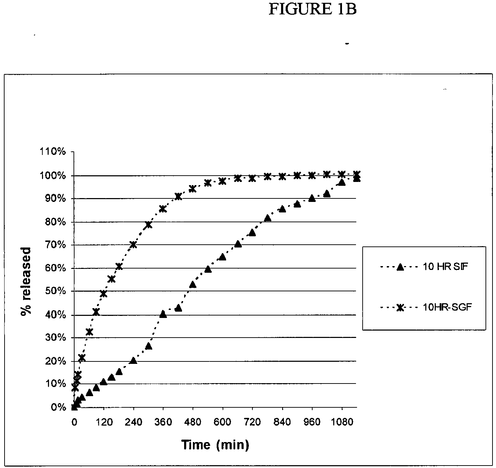

Figure 1 is the dissolution profile of the composition of Example 1 in simulated gastric fluid (SGF) and simulated intestinal fluid (SIF). This is a comparative example, not of the invention.

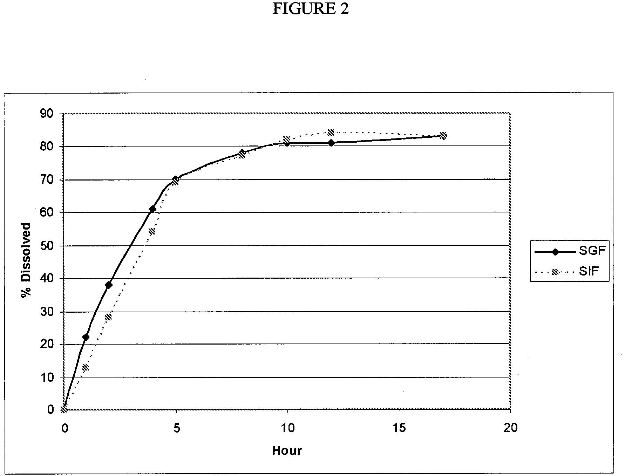

Figure 2 is the *in vitro* dissolution profile of the matrix tablet composition of Example 2 in simulated gastric fluid (SGF) and simulated intestinal fluid (SIF).

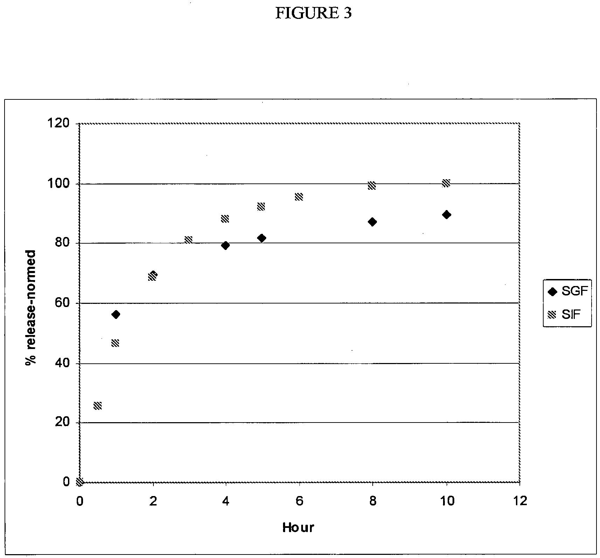

Figure 3 is the *in vitro* dissolution profile of the matrix pellet composition of Example 3 in simulated gastric fluid (SGF) and simulated intestinal fluid (SIF).

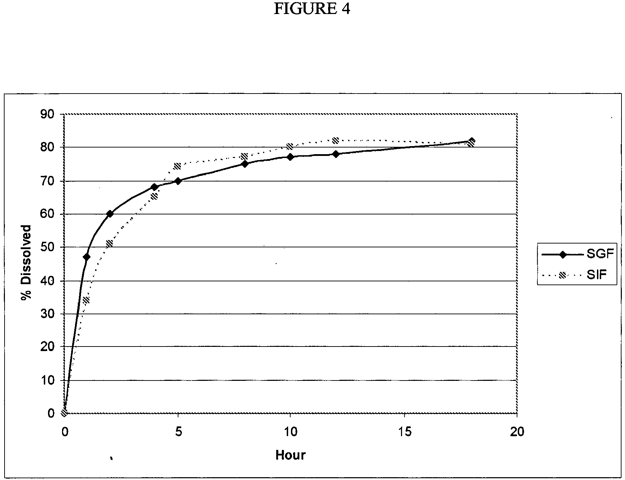

Figure 4 is the *in vitro* dissolution profile of the matrix pellet composition of Example 4 in simulated gastric fluid (SGF) and simulated intestinal fluid (SIF).

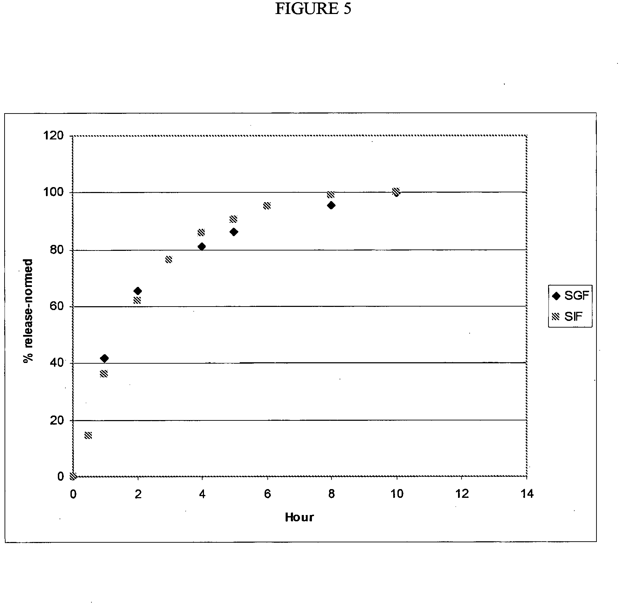

Figure 5 is the *in vitro* dissolution profile of the layered pellet composition of Example 5 in simulated gastric fluid (SGF) and simulated intestinal fluid (SIF).

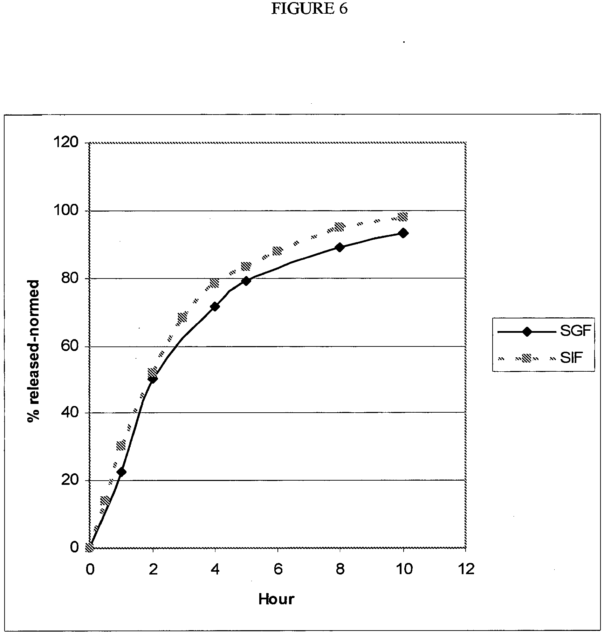

Figure 6 is the *in vitro* dissolution profile of the layered pellet composition of Example 6 in simulated gastric fluid (SGF) and simulated intestinal fluid (SIF).

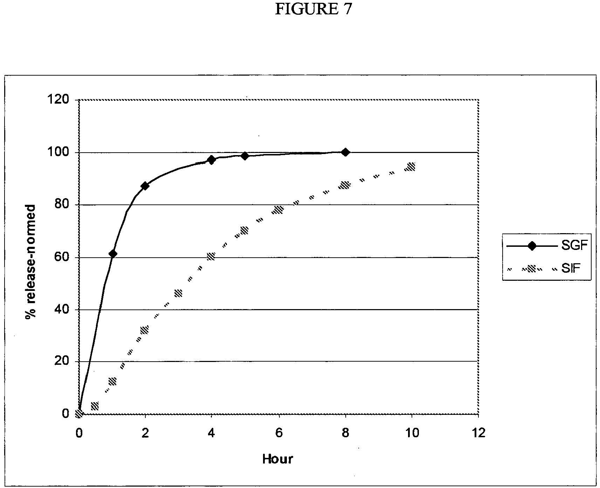

Figure 7 is the *in vivo* plasma dissolution profile and PK parameter of the compositions prepared in Example 7 in simulated gastric fluid (SGF) and simulated intestinal fluid (SIF) in the monkey.
This is a comparative example, not of the invention.

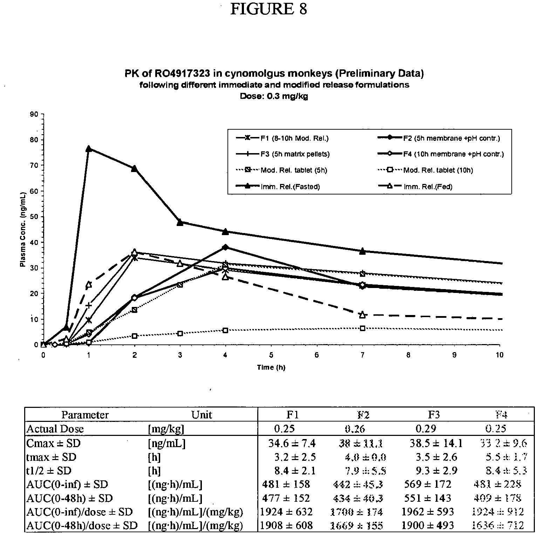

Figure 8 is the *in vivo* intrinsic dissolution PK profile of the composition in Example 1 (F6 and F7), Example 3 (F3), Example 5 (F2), Example 6 (F4), Example 7 (F1).

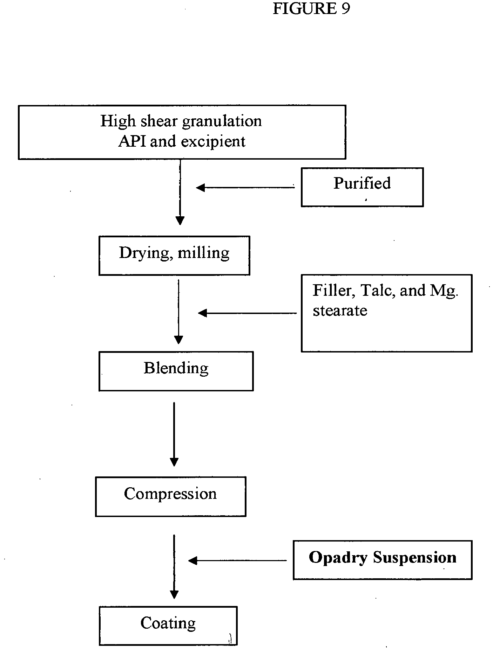

Figure 9 is a flow diagram depicting the process for preparing matrix tablet compositions disclosed herein.

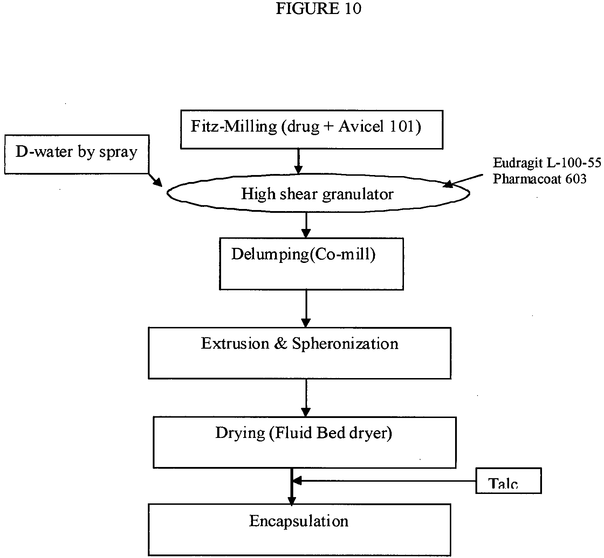

Figure 10 is a flow diagram depicting the process for preparing matrix pellet compositions disclosed herein.

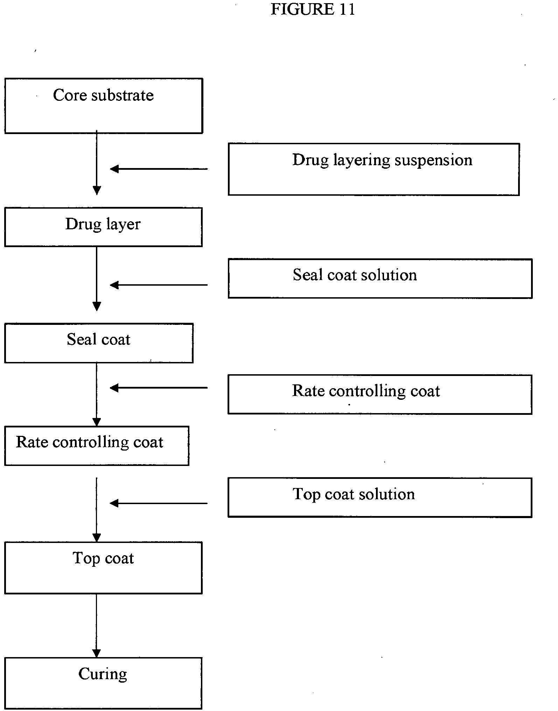

Figure 11 is a flow diagram depicting the process for preparing layered pellet compositions disclosed herein.

The composition described herein is a modified release technology that provides pH independent delivery of poorly water soluble drugs, particularly the metabotrobic glutatmate 5 receptor (mGlu5) antagonists of formula I. These compositions are in the form of matrix tablets, matrix pellets, or layered pellets, and each can be formed into tablets or incorporated in capsules. The present modified release formulations reduce CNS related adverse effects, improve therapeutic efficacy, improve tolerability, and reduce or eliminate food effect.

"Aryl" represents an aromatic carbocyclic group consisting of one individual ring, or one or more fused rings in which at least one ring is aromatic in nature. Preferred aryl group is phenyl.

The term "binder" refers to a substance used in the formulation of solid oral dosage forms to hold the active pharmaceutical ingredient and inactive ingredients together in a cohesive mix. Nonlimiting examples of binders include gelatin, hydroxy propyl cellulose, hydroxy propyl methylcellulose, methylcellulose, polyvinyl pyrrolidone, sucrose, and starch.

The term "cycloalkyl" denotes a saturated carbocyclic group, containing 3 - 12 carbon atoms, preferably 3-6 carbon atoms.

The term "disintegrant" refers to an excipients which is added to a tablet or capsule blend to aid in the break up of the compacted mass when it is put into a fluid environment. Non limited examples of disintegrants include alginates, croscarmellose sodium, crospovidone, sodium starch glycolate, and pregelatinized starch.

The term "filler" refers to any pharmaceutical diluent.

The term "gel-forming cellulose ethers" refers to polymers derived by chemical modification of the natural polymer cellulose that is obtained from renewable botanical sources that formS a gel in aqueous medium under certain conditions

The term "glidant" refers to a substance that is added to a powder to improve its flowability. Nonlimiting examples of glidants include colloidal silicon dioxide, magnesium stearate, starch, and talc.

The term "halogen" denotes fluorine, chlorine, bromine and iodine.

The term "heteroaryl" refers to an aromatic 5- or 6-membered ring containing one or more heteroatoms selected from nitrogen, oxygen or sulphur. Preferred are those heteroaryl groups selected from nitrogen. Examples of such heteroaryl groups are pyridinyl, pyrazinyl, pyrimidinyl or pyridazinyl.

The term "hydrophilic polymers" refers to polymers that contain polar or charged functional groups, rendering them soluble in aqueous medium.

The term "insoluble polymer" refers to a polymer that is not soluble in aqueous medium.

The term "ionic polymer" refers to a polymer that consists of functional groups that are sensitive to pH. Depending on the pH, functional groups can ionize and help dissolve the polymer. "Anionic polymers" as used herein are generally soluble above about pH 5.

The term "lower alkyl" used in the present description denotes straight-chain or branched saturated hydrocarbon residues with 1 to 6 carbon atoms, preferably with 1 to 4 carbon atoms, such as methyl, ethyl, n-propyl, i-propyl, n-butyl, t-butyl and the like.

The term "lower alkoxy" denotes a lower alkyl residue in the sense of the foregoing definition bound via an oxygen atom. Examples of "lower alkoxy" residues include methoxy, ethoxy, isopropoxy and the like.

The term "lower haloalkoxy" denotes lower alkoxy group as defined above which is substituted by one or more halogen. Examples of lower haloalkoxy include but are not limited to methoxy or ethoxy, substituted by one or more Cl, F, Br or I atom(s) as well as those groups specifically illustrated by the examples herein below. Preferred lower haloalkoxy are difluoro- or trifluoro-methoxy or ethoxy.

The term "lower haloalkyl" denotes a lower alkyl group as defined above which is substituted by one or more halogen. Examples of lower haloalkyl include but are not limited to methyl, ethyl, propyl, isopropyl, isobutyl, sec-butyl, tert-butyl, pentyl or n-hexyl substituted by one or more Cl, F, Br or I atom(s) as well as those groups specifically illustrated by the examples herein below. Preferred lower haloalkyl are difluoro- or trifluoro-methyl or ethyl.

The term "lubricant" refers to an excipients which is added to a powder blend to prevent the compacted powder mass from sticking to the equipment during the tabletting or encapsulation process. It aids the ejection of the tablet form the dies, and can improve powder flow. Nonlimiting examples of lubricants include calcium stearate, glycerine, hydrogenated vegetable oil, magnesium stearate, mineral oil, polyethylene glycol, and propylene glycol.

The term "matrix former" refers to a nondisintegrating polymer that provides the rigidity or we mechanical strength to the dosage form upon exposure to physiological fluid for controlling the release.

The term "modified-release" technology is the same as sustained-release (SR), sustained-action (SA), extended-release (ER, XR, or XL), timed-release, controlled-release (CR), and refers of a technology that provide release of a drug from a formulation over a defined period of time.

The term "multi-particulate composition" refers to solid particle systems employed in drug delivery systems including pellets, beads, millispheres, microspheres, microcapsules, aggregated particles, and others.

The term "particle size" refers to a measure of the diameter of the material as determined by laser diffraction.

The term "pH responding polymer" refers to ionizable polymers with pH dependent solubility that change permeability in response to changes in the gastrointestinal tract's physiological pH. Nonlimiting examples of pH responding polymers include hydroxypropylmethyl cellulose phthalate, cellulose acetate phthalate, cellulose acetate trimellitate, poly(meth)acrylates, and mixtures thereof. In one embodiment, poly(meth)acrylate.

The term "pharmaceutically acceptable," such as pharmaceutically acceptable carrier, excipient, etc. means pharmacologically acceptable and substantially non-toxic to the subject to which the particular compound is administered.

The term "pharmaceutically acceptable salt" refers to any salt derived from an inorganic or organic acid or base. Such salts include: acid addition salts formed with inorganic acids such as hydrochloric acid, hydrobromic acid, sulfuric acid, nitric acid, phosphoric acid; or formed with organic acids such as acetic acid, benzenesulfonic acid, benzoic, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid or trimethylacetic acid.

The term "plasticizer" refers to a substance that reduces the glass transition temperature of a polymer, making it more elastic and deformable, i.e. more flexible. Nonlimiting examples of plasticizers include dibutylsebacate, propylene glycol, triethylcitrate, tributylcitrate, castor oil, acetylated monoglycerides, acetyl triethylcitrate, acetyl butylcitrate, diethyl phthalate, dibutyl phthalate, triacetin, and medium-chain triglycerides.

The term "poorly soluble" refers to a compound whose solubility is below 33 mg/ml.

The term "rate controlling polymer" refers to pH independent, insoluble polymers that provide a pH independent permeability for drug release in a rate-controlling polymer membrane.

The term "release modifier" refers to any material that ca change the dissolution rate of the active ingredient when added to the composition.

The term "spheronization enhancer" refers to a material added to the composition to enhance the sphericity of the particles in the composition.

The term "substantially water-soluble inert material" refers to any material that has a solubility in water greater than about 1 % w/w.

The term "surfactant" refers to a surface active compound that lowers the surface tension of a liquid and lowers the interfacial tension between two liquids, or between a liquid and a solid. Nonlimiting examples of surfactants include polysorbates and sodium lauryl sulfate.

The term "weakly basic" refers to compounds that are freely to moderately soluble at acidic pHs, but are poorly to practically insoluble at neutral and alkaline pHs, using the USP definitions for solubility.

The layered pellet compositions comprise a modified release core that is coated with a pH responding modified enteric coat. The combination of the controlled release core and the pH responding coat allow drug release to begin in the stomach without delaying onset of drug and to continue at a sustained rate over a period of approximately 10 hours. The combination of the rate controlling polymer and pH responding polymer enable continuous drug release in the gastric fluid without shutting down or delaying drug release. This release profile provides continuous release of the drug for absorption without the risk of dose dumping, which is generally associated with an enteric polymer coating due to variations in gastic pH and transit time. After gastric transition, the pH increases to from about 5.5 to about 7, resulting in a decrease in solubility of the basic compound of formula I. The pH responding polymer swells and dissolves, providing increased film permeability that compensates for the decrease in drug solubility, which enables a pH independent release rate.

The matrix tablet and matrix pellet utilize a combination of pH responding enteric polymer and a rate controlling polymer as matrix components. The enteric polymer provides a pH microenvironment that results in a constant concentration gradient for drug diffusion through the matrix layer. After gastric transition, the pH increases to from about 5.5 to about 7, resulting in a decrease in solubility of the basic compound of formula I. The pH responding polymer swells and dissolves, causing an increase in matrix porosity that compensates for the decrease in drug solubility, which enables a pH independent release rate.

The amount of the mGlu5 antagonist in the composition can vary from about 0.005 % to about 5 % by weight of the composition. In one embodiment, the amount of mGlu5 antagonist is from about 0.05 % to about 5 % by weight of the composition. In another embodiment the amount of mGlu5 antagonist is from about 0.005 % to about 0.5 % of the composition.

The particle size of the mGlu5 antagonist is ideally reduced to below 50 micron. In one embodiment the particle size of the compound is reduced to below 20 micron. In another embodiment, the particle size is reduced to below 10 micron (D90) for the mGlu5 antogonist

### Active Ingredient

The active ingredient of the compositions are metabotropic glutamate 5 receptor (mGlu5) antagonists. Such compounds, their methods of manufacture, and therapeutic activity are described in commonly owned U.S. Patent Publication No. 2006-0030559, published February 9, 2006, and U.S. Patent No. 7,332,510, issued February 19, 2008, each incorporated by reference herein.

In one embodiment, the metabotropic glutamate 5 receptor (mGlu5) antagonist comprises a compound of formula I 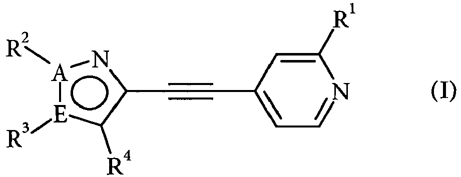 wherein
one of A or E is N and the other is C;
- R¹: is halogen or cyano;
- R²: is lower alkyl;
- R³: is aryl or heteroaryl, each of which is optionally substituted by one, two or three substituents chosen from halogen, lower alkyl, lower alkoxy, cycloalkyl, lower haloalkyl, lower haloalkoxy, cyano, or NR'R", or by 1-morpholinyl, 1-pyrrolidinyl, optionally substituted by (CH₂)ₘOR, piperidinyl, optionally substituted by (CH₂)ₘOR, 1,1 -dioxo-thiomorpholinyl, or piperazinyl, optionally substituted by lower alkyl or (CH₂)ₘ-cycloalkyl;
- R: is hydrogen, lower alkyl or (CH₂)ₘ-cycloalkyl;
- R' and R": are each independently hydrogen, lower alkyl, (CH₂)ₘ-cycloalkyl or (CH₂)ₙOR;
- m: is 0 or 1;

- n: is 1 or 2; and
- R⁴: is CHF₂, CF₃, C(O)H, or CH₂R⁵, wherein R⁵ is hydrogen, OH, C₁-C₆-alkyl or C₃-C₁₂-cycloalkyl;
and pharmaceutically acceptable salts thereof.

In one embodiment, the compound of formula I can have the formula Ia 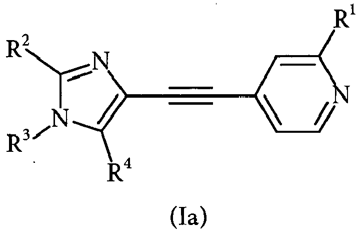 wherein R¹, R², R³ and R⁴ are as defined herein above.

In another embodiment, compounds of formula Ia, comprise those wherein R³ is unsubstituted or substituted heteroaryl, wherein the substitution is selected from chloro, fluoro, CF₃, and lower alkyl, for example the following compounds:
2-[4-(2-Chloro-pyridin-4-ylethynyl)-2,5-dimethyl-1H-imidazol-1-yl]-5-methyl-pyridine;
2-Chloro-5-[4-(2-Chloro-pyridin-4-ylethynyl)-2,5-dimethyl-1H-imidazol-1-yl]-pyridine;
2-[4-(2-Chloro-pyridin-4-ylethynyl)-2,5-dimethyl-1H-imidazol-1-yl]-6-methyl-4-trifluoromethyl-pyridine;
2-[4-(2-Chloro-pyridin-4-ylethynyl)-2,5-dimethyl-1H-imidazol-1-yl]-pyrazine;
2-[4-(2-Chloro-pyridin-4-ylethynyl)-2,5-dimethyl-1H-imidazol-1-yl]-6-methyl-pyridine;
2-[4-(2-Chloro-pyridin-4-ylethynyl)-2,5-dimethyl-1H-imidazol-1-yl]-6-(trifluoromethyl)-pyridine; and
3-[4-(2-Chloro-pyridin-4-ylethynyl)-2,5-dimethyl-1H-imidazol-1-yl]-5-fluoro-pyridine.

In yet another embodiment, compounds of formula Ia comprise those wherein R³ is aryl, substituted by one, two, or three chloro, fluoro, CF₃, lower alkyl, lower alkoxy, CF₃O, and 1-morpholinyl, for example the following compounds:
2-Chloro-4-[1-(4-fluoro-phenyl)-2,5-dimethyl-1H-imidazol-4-ylethynyl]-pyridine;
2-Chloro-4-[1-(2,4-difluoro-phenyl)-2,-dimethyl-1H-imidazol-4-ylethynyl]-pyridine;
2-Chloro-4-[1-(3,5-difluoro-phenyl)-2,5-dimethyl-1H-imidazol-4-ylethynyl]-pyridine;
2-Chloro-4-[1-(4-fluoro-2-methyl-phenyl)-2,5-dimethyl-1H-imidazol-4-ylethynyl]-pyridine;
2-Chloro-4-[1-(4-fluoro-3-methyl-phenyl)-2,5-dimethyl-1H-imidazol-4-ylethynyl]-pyridine;
2-Chloro-4-(2,5-dimethyl-1-p-tolyl-1H-imidazol-4-ylethynyl)-pyridine;
2-Chloro-4-[1-(3-chloro-4-methyl-phenyl)-2,5-dimethyl-1H-imidazol-4-ylethynyl]-pyridine;
2-Chloro-4-[1-(3-fluoro-4-methoxy-phenyl)-2,5-dimethyl-1H-imidazol-4-ylethynyl]-pyridine;
2-Chloro-4-[1-(4-methoxy-phenyl)-2,5-dimethyl-1H-imidazol-4-ylethynyl]-pyridine;
2-Chloro-4-[2,5-dimethyl-1-(4-trifluoromethoxy-phenyl)-1H-imidazol-4-ylethynyl]-pyridine;
2-Chloro-4-[2,5-dimethyl-1-(3-trifluoromethoxy-phenyl)-1H-imidazol-4-ylethynyl]-pyridine;
2-Chloro-4-[2,5-dimethyl-1-(4-trifluoromethyl-phenyl)-1H-imidazol-4-ylethynyl]-pyridine;
2-Chloro-4-[2,5-dimethyl-1-(3-methyl-4-trifluoromethoxy-phenyl)-1H-imidazol-4-ylethynyl]-pyridine;
2-Chloro-4-[1-(4-chloro-phenyl)-2,5-dimethyl-1H-imidazol-4-ylethynyl]-pyridine;
2-Chloro-4-[1-(3-chloro-2-fluoro-phenyl)-2,5-dimethyl-1H-imidazol-4-ylethynyl]-pyridine;
2-Chloro-4-[2,5-dimethyl-1-(3-trifluoromethyl-phenyl)-1H-imidazol-4-ylethynyl]-pyridine;
2-Chloro-4-[1-(3-chloro-4-fluoro-phenyl)-2,5-dimethyl-1H-imidazol-4-ylethynyl]-pyridine;
2-Chloro-4-[2,5-dimethyl-1-(2-methyl-4-trifluoromethoxy-phenyl)-1H-imidazol-4-ylethynyl]-pyridine;
2-Chloro-4-[5-difluoromethyl-1-(4-fluoro-phenyl)-2-methyl-1H-imidazol-4-ylethynyl]-pyridine;
[5-(((2-Chloro-pyridin-4-ylethynyl)-3-(4-fluoro-phenyl)-2-methyl-3H-imidazol-4-yl]-methanol;
2-Chloro-4-[1-(4-methoxy-3-trifluoromethyl-phenyl)-2,5-dimethyl-1H-imidazol-4-ylethynyl]-pyridine;
2-Chloro-4-[1-(3,5-difluoro-4-methoxy-phenyl)-2,5-dimethyl-1H-imidazol-4-ylethynyl]-pyridine;
2-Chloro-4-[1-(4-methoxy-3-trifluoromethoxy-phenyl)-2,5-dimethyl-1H-imidazol-4-ylethynyl]-pyridine;
2-Chloro-4-[1-(3-methoxy-4-trifluoromethoxy-phenyl)-2,5-dimethyl-1H-imidazol-4-ylethynyl]-pyridine;
4-{3-[4-(2-Chloro-pyridin-4-ylethynyl)-2,5-dimethyl-imidazol-1-yl]-5-fluoro-phenyl}-morpholine;
2-Chloro-4-[1-(4-fluoro-2-trifluoromethoxy-phenyl)-2,5-dimethyl-1H-imidazol-4-ylethynyl]-pyridine;
2-Chloro-4-[1-(2-fluoro-4-trifluoromethoxy-phenyl)-2,5-dimethyl-1H-imidazol-4-ylethynyl]-pyridine;
2-Chloro-4-[2,5-dimethyl-1-(4-methyl-3-trifluoromethyl-phenyl)-1H-imidazol-4-ylethynyl]-pyridine;
2-Chloro-4-[2,5-dimethyl-1-(3-methyl-4-trifluoromethyl-phenyl)-1H-imidazol-4-ylethynyl]-pyridine;
2-Chloro-4-[2,5-dimethyl-1-(3-methyl-5-trifluoromethyl-phenyl)-1H-imidazol-4-ylethynyl]-pyridine;
2-Chloro-4-[1-(3-methoxy-5-trifluoromethyl-phenyl)-2,5-dimethyl-1H-imidazol-4-ylethynyl]-pyridine;
2-Chloro-4-[1-(3-methoxy-4-trifluoromethyl-phenyl)-2,5-dimethyl-1H-imidazol-4-ylethynyl]-pyridine;
2-Chloro-4-[1-(3,5-dichloro-phenyl)-2,5-dimethyl-1H-imidazol-4-ylethynyl]-pyridine;
2-Chloro-4-[1-(3-chloro-5-methyl-phenyl)-2,5-dimethyl-1H-imidazol-4-ylethynyl]-pyridine;
2-Chloro-4-[1-(3-fluoro-5-methyl-phenyl)-2,5-dimethyl-1H-imidazol-4-ylethynyl]-pyridine;
2-Chloro-4-[1-(3-chloro-5-methoxy-phenyl)-2,5-dimethyl-1H-imidazol-4-ylethynyl]-pyridine; and
2-Chloro-4-[1-(3-fluoro-5-methoxy-phenyl)-2,5-dimethyl-1H-imidazol-4-ylethynyl]-pyridine.

In one embodiment, the compound of formula I can have the formula Ib 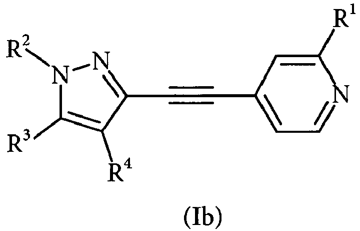 wherein R¹, R², R³ and R⁴ are as defined herein above.

In another embodiment, compounds of formula Ib comprise those wherein R³ is aryl, substituted by one, two or three fluoro, for example the compound 2-chloro-4-[5-(4-fluoro-phenyl)-1,4-dimethyl-1H-pyrazol-3-ylethynyl]-pyridine.

Non-limiting examples of pharmaceutically acceptable salts are organic acid addition salts formed with acids, which form a physiological acceptable anion, for example, tosylate, methanesulfonate, maleate, malate, acetate, citrate, malonate, tartarate, succinate, benzoate, ascorbate, α-ketoglutarate, and α-glycerophosphate. Other pharmaceutically acceptable salts include inorganic salts, such as, for example, hydrochloride, sulfate, nitrate, bicarbonate, and carbonate salts. In one embodiment, the salt form of the mGlu5 antagonist of formula I exhibits low hygroscopic and good aqueous solubility. In another embodiment the salt is sulphate.

Compounds of formula I have metabotropic glutamate 5 receptor (mGlu5) antagonist activity. They are useful for the treatment of CNS disorders, including, but not limited to, treatment-resistant depression (TRD) and Fragile X Syndrome. One such compound, 2-chloro-4-[1-(4-fluoro-phenyl)-2,5-dimethyl-1H-imidazol-4-ylethynyl]-pyridine, is typical of those of formula I and will be used to describe the compositions. It should be understood that the all compounds of formula I can be employed in the compositions described herein. The compound 2-chloro-4-[1-(4-fluoro-phenyl)-2,5-dimethyl-1H-imidazol-4-ylethynyl]-pyridine, has two weakly basic moieties with pKa values of 4.64 and approximately 2. The compound is very lipophilic with a clog P value of 3.71 and a log D at pH 7.4 of greater than 3. The aqueous solubility of the free base is characterized by a steep pH-dependency with good solubility under acidic conditions (3.2 mg/ml at pH 1) and very low solubility at alkaline conditions (0.0003 mg./ml at pH 7). Because of this pH dependent solubility in physiological range, 2-chloro-4-[1-(4-fluoro-phenyl)-2,5-dimethyl-1H-imidazol-4-ylethynyl]-pyridine is classified as BCS class 2 compound.

Due to high solubility in gastric pH, conventional immediate release (IR) formulations of the compounds of formula I provide rapid release of the active ingredient once the formulation reaches the stomach. The peak plasma concentration occurs one hour following drug administration. However, the disadvantage of these IR formulations is that CNS-related adverse events, such as dizziness and somnolence, occur. These adverse events appear to be associated with high plasma peak or with the fast rise in plasma concentration that occurs after administration of the drug. Moreover, significant food effect was observed with the IR formulation. Administration of IR formulations of the drug with food caused a reduction in peak plasma concentration and a delay in Tmax. Administration of the IR formulation with food also resulted in a better safety profile.

The present modified release formulations reduce CNS related adverse effects, improve therapeutic efficacy, improve tolerability, and reduce or eliminate food effect.

### Matrix Tablet

In one embodiment, the composition comprises a matrix type composition, e.g. a matrix tablet, where the drug, for example a compound of formula I, is dispersed in a rate controlling polymer. One type of rate controlling polymer is a hydrophilic polymer, for example, polyvinyl pyrrolidone, hydroxypropyl cellulose, hydroxypropylmethyl cellulose (HPMC), methyl cellulose, ethyl cellulose, vinyl acetate/crotonic acid copolymers, poly(meth)acrylates, maleic anhydride/methyl vinyl ether copolymers, polyvinylacetate/povidone copolyemrs, and derivatives and mixtures thereof. The mechanism of release from these matrices depends on the aqueous solubility of the drug and the hydrophilicity of the polymer used. In another embodiment the hydrophilic polymer is a gel-forming cellulose ether. Nonlimiting examples of gel-forming cellulose ethers that can be used are hydroxypropyl cellulose and hydroxypropylmethyl cellulose.

In another embodiment, HPMC (K100 LV and K100M) can be selected as the rate controlling polymer. The amount of the rate controlling polymer, for example HPMC, in the composition can vary from about 5 % to about 50 % by weight of the composition. In one embodiment, the rate controlling polymer can be present in an amount from about 10 % to about 35 % by weight of the composition. In another embodiment, the rate controlling polymer can be present in an amount from about 10 % to about 25 % of the composition.

The matrix tablet also can comprise other ingredients, such as fillers, surfactants, glidants, lubricants, and/or binders that are commonly used for tablet composition. Such ingredients include, for example, lactose monohydrate, microcrystalline cellulose (Avicel PH 102^{®}), corn starch, calcium hydrogen phosphate anhydrous (Fujicalin^{®}), mannitol, polyvidone (Povidone K30^{®}), hydroxypropyl methylcellulose (HPMC 2910^{®}), magnesium stearate, sodium stearyl fumarate, stearic acid, colloidal silicon dioxide (AEROSIL 200^{®}), gelatin, polyoxypropylene-polyoxyethylene copolymer (Pluronic F68^{®}), sodium dodecyl sulfate (SDS), sucrose mono palmitate (D1616), polyethylene glycol (40) monostearate (Myrj 52^{®}), talc, titanium dioxide, such as microcrystalline cellulose (MCC), lactose, polyvinayl chloride (PVC), and sodium starch glycolate.

The present composition also includes an ionizable, pH responding polymer. This polymer can overcome the drawbacks associated with the use of a hydrophilic polymer for weakly basic compounds. Because the rate of release might be dependent upon drug solubility in the gastrointestinal environment, incorporation of such additional polymers assists in creating a rate of release that is independent of the pH. Such pH responding polymers provide a pH micro-environment that imparts a constant concentration gradient for drug diffusion through the matrix or gel layer. After gastric transition, the pH increases to from about 5.5 to about 7, and the solubility of the basic mGlu5 antagonist decreases. In response to these conditions, the pH-responding enteric polymer swells and dissolves causing an increase in matrix porosity that compensates for the decrease in drug solubility and enables a pH independent release rate.

pH responding polymers include, but are not limited to, hydroxypropylmethyl cellulose phthalate, cellulose acetate phthalate, hydroxypropylmethyl cellulose acetate succinate, cellulose acetate trimellitate, ionic poly(meth)acrylates, polyvinyl phthalate, and mixtures thereof. In one embodiment, poly(meth)acrylate (e.g., Eudragit L100-55^{®} or Eudragit L100^{®}) can be used to prepare the matrix compositions herein. In one embodiment, a poly(meth)acrylate, such as Eudragit L100-55^{®}, can be selected as the pH responding polymer for the matrix tablet described herein with a salt or derivative of a compound of formula I. The amount of pH responding polymer in the composition can be from about 5 % to about 50 % by weight of the composition. In one embodiment, the pH responding polymer can be present in an amount from about 10 % to about 35 % by weight of the composition. In a further embodiment, pH responding polymer can be present in an amount from about 10 % to about 25 % of the composition. The composition exhibits an *in-vitro* release profile with not more than (NMT) 70 % in one hour, NMT 85 % in 4 hour, and not less than (NLT) 80 % in 8 hours.

In one embodiment, the composition comprises 2-chloro-4-[1-(4-fluoro-phenyl)-2,5-dimethyl-1H-imidazol-4-ylethynyl]-pyridine, HPMC, Eudragit L100-55, and other conventional excipients. Such a composition can provide pH independent, controlled delivery of the compound with a reduction of Cmax and absorption rate over conventional compositions employing a hydrophilic HPMC polymer.

The pH responding polymer also can be an insoluble polymer and can be used in combination with or without hydrophilic polymers. The mechanism of drug release for a matrix tablet containing an insoluble polymer is to modulate the permeability of the matrix. The aqueous fluids, e.g. gastrointestinal fluids, penetrate and dissolve the drug, which can then diffuse out from the matrix. The rate of release is dependent upon the permeability of the matrix and the solubility of the drug in the gastrointestinal environment. Nonlimiting examples of such insoluble polymers include ethyl cellulose (EC) and polyvinylacetate. In one embodiment, the insoluble polymer is ethyl cellulose (EC) or polyvinylacetate. In another embodiment, the insoluble polymer is polyvinylacetate.

The amount of insoluble polymer in the composition can vary from about 5 % to about 50 % by weight of the composition. In one embodiment, the insoluble polymer can be present in an amount from about 10 % to 35 % by weight of the composition. In another embodiment, the insoluble polymer can be present in an amount from about 10% to about 25 % by weight of the composition. The composition exhibits an *in-vitro* release profile with NMT 70 % in one hour, NMT 85 % in 4 hour, and NLT 80 % in 8 hours.

Matrix tablet formulations can be manufactured by a series of processes known in the art, for example, by wet granulation, drying, milling, blending, compression, and film-coating. (See, e.g., Robinson and Lee, Drugs and Pharmaceutical Sciences, Vol. 29, Controlled Drug Delivery Fundamentals and Applications and US Patent 5,334,392). In general, the drug and polymer mixture is granulated to obtain a uniform matrix of drug and polymer. This consolidates the particle and improves flow. The granulated product is then dried to remove moisture and milled to deagglomerate the product. The product is then blended to obtain a uniform mix and lubricant is added to eliminate sticking of the matrix to the die wall and punch surfaces during formation of the tablets. Next, the product is compressed into tablets that are coated with a film-coat to improve surface characteristics, improve the ease with which the product can be swallowed, and mask any undesirable taste.

### Matrix Pellets

In one embodiment, the composition comprises matrix pellets, where drug, for example the mGlu5 antagonist of formula I, is dispersed in the composition that is formed into pellets. The matrix pellets optionally can be coated with a further polymer layer and optionally encapsulated into capsules or compressed into tablets. In general, the drug and excipients are blended to form a uniform mixture. The mixture is then granulated to obtain a uniform matrix of drug and polymer. This consolidates the particle and improves flow. The granulated product is then extruded and then spheronized to form dense pellets having a spherical shape. The pellets then are dried to remove moisture.

The matrix pellet composition can be manufactured by methods known in the art, for example by extrusion spheronization, rotor granulation, spray drying, hot melt extrusion, top granulation and other standard technologies. In one embodiment, extrusion spheronization can be selected as the technology for manufacturing the matrix pellets. (See, e.g., Trivedi et al, Critical Reviews in Therapeutic Drug Carrier Systems, 24(1): 1-40 (2007); US Patent 6,004,996; and Issac-Ghebre-Selassi, et al., eds. Durgs and Pharmaceutical Sciences, Vol. 133, Pharmaceutical Extrusion Technology.)

Excipients can be used in the extrusion/spheronization process. These excipients can be selected based on functionality of the excipients. Nonlimiting examples of types of excipients that can be used include fillers, binders, lubricants, disintegrants, surfactants, spheronization enhancers, glidants, and release modifiers. Some nonlimiting examples of each of these types of excipients follows. Fillers can include, for example, calcium sulfate, dibasic calcium phosphate, lactose, mannitol, microcrystalline cellulose, starch, and sucrose. Binders can include, for example, gelatin, hydroxy propyl cellulose, hydroxy propyl methylcellulose, methylcellulose, polyvinyl pyrrolidone, sucrose, and starch. Lubricants can include, for example, calcium stearate, glycerine, hydrogenated vegetable oil, magnesium stearate, mineral oil, polyethylene glycol, and propylene glycol. Disintegrants can include, for example, alginates, croscarmellose sodium, crospovidone, sodium starch glycolate, and pregelatinized starch. Surfactants can include, for example, polysorbates and sodium lauryl sulfate. Spheronization enhancers can include, for example, microcrystalline cellulose, microcrystalline, and cellulose/sodium-carboxymethyl cellulose. Glidants can include, for example, colloidal silicon dioxide, magnesium stearate, starch, and talc. Release modifiers can include, for example, ethyl cellulose, carnauba wax, and shellac.

In one embodiment, the matrix pellets contain MCC as a matrix former, HPMC as binder, and alternatively, an ionizable, pH responding polymer. The pH responding polymer can be any of those described above. In one embodiment, the pH responding polymer can be an ionic polymer, such as a poly(meth)acrylate like Eudragit L100-55^{®}. As discussed above, such pH responding polymers overcome the pH dependency of drug release for weakly basic compounds, such as those used in the described matrix pellets. As with the matrix tablet, the pH responding polymer creates a pH micro-environment that provides a constant concentration gradient for drug diffusion through the matrix or gel layer of the matrix pellet. After gastric transition, the pH increases to from about 5.5 to about 7 and the solubility of the basic mGlu5 antagonist decreases. In response to these conditions, the pH-responding enteric polymer swells and dissolves causing an increase in matrix porosity that compensates the decrease in drug solubility and enables a pH independent release rate.

The amount of pH responding polymer in the composition can vary from about 5 % to about 50 % by weight of the composition. In one embodiment, the pH responding polymer can be present in an amount from about 10 % to about 40 % by weight of the composition. In another embodiment, the pH responding polymer can be present in an amount from about 25 % to about 35 % of the composition. The composition exhibits an *in-vitro* release profile with NMT 70 % in one hour, NMT 85 % in 4 hour, and NLT 80 % in 8 hours.

In one embodiment, the particle size of the matrix pellet comprising the mGlu5 antagonist is ideally below about 3000 microns. In another embodiment, the particle size of the pellet is below about 2000 microns. In yet another embodiment, the average particle size of the pellet is about 400 microns to about 1500 microns.

The pH responding polymer also can be an insoluble polymer and can be used in combination with or without hydrophilic polymers. The mechanism of drug release for a matrix tablet containing an insoluble polymer is to modulate the permeability of the matrix. The aqueous fluids, e.g. gastrointestinal fluids, penetrate and dissolve the drug, which can then diffuse out from the matrix. Examples of insoluble polymers include, but are not limited to, ethyl cellulose (EC), polyvinylacetate (Kollidon SR^{®}), and polyvinylacetate/povidone copolymer. In one embodiment, ethyl cellulose (EC) or polyvinylacetate can be used to prepare the matrix pellets. In another embodiment, polyvinylacetate can be used to prepare the matrix pellets.

The amount of insoluble polymer in the composition can vary from about 5 % to about 50 % by weight of the composition. In one embodiment, the insoluble polymer can be present in an amount from about 10 % to about 3 5% by weight. In another embodiment, the insoluble polymer can be present in an amount from about 5 % to about 25 % of the composition. The composition exhibits an *in-vitro* release profile with NMT 70 % in one hour, NMT 85 % in 4 hour, and NLT 80 % in 8 hours.

### Layered Pellets

Layered pellets comprise a drug-loaded discrete pellet core covered with polymer coating. They can be manufactured by methods known in the art, for example, rotor granulation, spray coating, Wurster coating, and other standard technologies. In one embodiment, a fluid bed Wurster coating process can be selected as the technology for manufacturing the layered pellets. Optionally, the layered pellets can be further compressed into tablets or be incorporated in a capsule (See, e.g., for conventional process US Patent 5,952,005). In general, drug is formulated into a polymer and loaded into an inert core material. The core material is then coated with one or more polymeric coating that modify drug release or modify the properties of the particle, e.g., reduce agglomeration. The pellets then are cured to provide a uniform coating and to reduce batch to batch variation.

The layered pellets comprise an inert core, such as a sugar spheres, microcrystalline cellulose beads, and starch beads. The inert core is coated with an inner drug-containing layer, a rate controlling layer that controls drug release from the inner layer, and a layer containing a pH responding polymer. Optionally, the layered pellet can include additional layers between the inner and outer layer and on top of the outer rate controlling layer.

In one embodiment the layered pellet comprises the following layers:
(i) a core unit of a substantially water-soluble or water-swellable inert material, for example, sugar spheres, microcrystalline cellulose beads, and starch beads.
(ii) a first layer covering the core, which contains an active ingredient, i.e. an mGlu5 antagonist; and
(iii) optionally, a second layer covering the first layer for separation of drug-containing layer and the rate controlling layer, and
(iv) a third controlled release layer, which contains a rate controlling polymer for controlled release of the active ingredient,
(v) a fourth layer containing a pH responding polymer for pH-independent controlled release of the active ingredient, and
(vi) optionally, a coating of a non-thermoplastic soluble polymer that decreases tackiness of the beads during curing and storage. Optionally, this coating layer can contain drug for immediate release.

In one embodiment, the core typically has a size in the range of from about 0.05 mm to about 2 mm; the first layer covering core constitutes from 0.005 % to 50 % of the final bead depending on the drug loading. In another embodiment, the first layer constitutes from about 0.01 % (w/w) to about 5 % (w/w).

In one embodiment, the amount of the second layer generally constitutes from about 0.5 % to about 25 % (w/w) of the final bead composition. In another embodiment, the amount of the second layer constitutes from about 0.5 % to about 5 % (w/w) of the final bead composition.

In one embodiment, the amount of the third layer generally constitutes from about 1 % to about 50 % (w/w). In anther embodiment, the amount of the third layer constitutes from about 5 % to about 15 % (w/w) of the final bead composition.

In one embodiment, the amount of the fourth layer generally constitutes from about 1 % to about 50 % (w/w). In another embodiment, the amount of the fourth layer constitutes from about 5 % to about 15 % (w/w) of the final bead composition.

In one embodiment, the amount of the coating generally constitutes from about 0.5 % to about 25 % (w/w). In another embodiment, the amount of the coating constitutes from about 0.5 % to about 5 % (w/w) of the final bead composition.

The core comprises a water-soluble or swellable material, and can be any such material that is conventionally used as cores or any other pharmaceutically acceptable water-soluble or water-swellable material made into beads or pellets. The cores can be, for example, spheres of materials such as sugar spheres, starch spheres, microcrystalline cellulose beads (Cellet^{®}), sucrose crystals, or extruded and dried spheres. The particle size of the pellet core is generally below about 3000 microns. In one embodiment, the particle size of the pellet core is below about 2000 microns. In another embodiment, the average particle size of the pellet core is from about 400 microns to about 1500 microns.

The first layer containing the active ingredient can be comprised of the active ingredient, i.e. an mGlu5 antagonist, with or without a polymer as a binder. The binder, when used, is typically hydrophilic but also can be water-soluble or water-insoluble. Exemplary polymers that can be incorporated in the first layer containing the active ingredient, e.g. a compound of formula I, are hydrophilic polymers. Nonlimiting examples of such hydrophilic polymers include polyvinylpyrrolidone (PVP), a polyalkylene glycol such as polyethylene glycol, gelatin, polyvinyl alcohol, starch and derivatives thereof, cellulose derivatives, such as hydroxypropylmethyl cellulose (HPMC), hydroxypropyl cellulose, carboxymethyl cellulose, methyl cellulose, ethyl cellulose, hydroxyethyl cellulose, carboxyethyl cellulose, and carboxymethylhydroxyethyl cellulose, acrylic acid polymers, poly(meth)acrylates, or any other pharmaceutically acceptable polymer. The ratio of drug to hydrophilic polymer in the second layer is generally in the range of from 1:100 to 100:1 (w/w).

The separation layer comprises a water soluble or permeable material. Exemplary polymers to be used in the separation layer are hydrophilic polymers such as polyvinylpyrrolidone (PVP), copovidone, a polyalkylene glycol such as polyethylene glycol, gelatin, polyvinyl alcohol, starch and derivatives thereof, cellulose derivatives, such as hydroxypropylmethyl cellulose (HPMC), hydroxypropyl cellulose, carboxymethyl cellulose, methyl cellulose, ethyl cellulose, hydroxyethyl cellulose, carboxyethyl cellulose, and carboxymethylhydroxyethyl cellulose, acrylic acid polymers, poly(meth)acrylates, or any other pharmaceutically acceptable polymer or mixtures thereof. In one embodiment, the separation layer is comprised of HPMC.

The third controlled release layer comprises a rate controlling polymer. The rate controlling polymer comprises a water-insoluble material, water swellable material, water soluble polymer, or any combination of these. Examples of such polymers include, but are not limited to, ethyl cellulose, polyvinylacetate, polyvinylacetate:povidone copolymer, cellulose acetate, poly(meth)acrylates such as ethyl acrylate/methyl methacrylate copolymer (Eudragit NE-30-D), and polyvinylacetate (Kollicoat SR, 30D^{®}). A plasticizer is optionally employed with the polymer. Exemplary plasticizers include, but are not limited to, dibutylsebacate, propylene glycol, triethylcitrate, tributylcitrate, castor oil, acetylated monoglycerides, fractionated coconut oil, acetyl triethylcitrate, acetyl butylcitrate, diethyl phthalate, dibutyl phthalate, triacetin, and medium-chain triglycerides. The controlled release layer optionally comprises another water soluble or swellable poreforming material to adjust the permeability, and thereby the release rate, of the controlled release layer. Exemplary polymers that can adjust permeability include HPMC, hydroxyethyl cellulose, hydroxypropyl cellulose, methylcellulose, carboxymethylcellulose, polyethylene glycol, polyvinylpyrrolidone (PVP), polyvinyl alcohol, polymers with pH-dependent solubility, such as cellulose acetate phthalate or ammonio methacrylate copolymer and methacrylic acid copolymer, or mixtures thereof. The controlled release layer also can include additional poreforming agents such as manitol, sucrose, lactose, sodium chloride. Pharmaceutical grade excipients also can be included in the controlled release layer, if desired.

The ratio of water-insoluble material, water swellable material, or water soluble polymer to permeability modifying agent in the third layer is typically in the range of from 100:0 to 1:100 (w/w).

The fourth layer comprises a pH responding polymer for controlling drug release. Nonlimiting examples of such pH responding polymers include hydroxypropylmethyl cellulose phthalate, cellulose acetate phthalate, cellulose acetate trimellitate, poly(meth)acrylates, or mixtures thereof. The pH responding polymer optionally can be combined with plasticizers, such as those mentioned above. The combination of the rate controlling layer and the pH responding layer enable continuous drug release in gastric fluid without shutting down or delaying drug release, which results in continuous release of drug for absorption without the risk of dose dumping associated with conventional enteric polymer coatings that results from inter and intra variation in gastric pH and transit time. After gastric transition, the pH increases to from about 5.5 to about 7 and the solubility of the basic mGlu5 antagonist decreases. In response to these conditions, the permeability of the pH-responding enteric polymer increases and compensates for the decrease in solubility of the mGlu5 antagonist and enables a pH independent release rate.

Optionally, the layer containing a pH responding polymer comprises another water soluble or swellable poreforming materials to adjust the permeability, and thereby the release rate, of the layer. Nonlimiting examples of polymers that can be used as a modifier together with, insoluble polymer include HPMC, hydroxyethyl cellulose, hydroxypropyl cellulose, methylcellulose, carboxymethylcellulose, polyethylene glycol, polyvinylpyrrolidone (PVP), polyvinyl alcohol, polymers with pH-dependent solubility, such as cellulose acetate phthalate or ammonio methacrylate copolymer and methacrylic acid copolymer, or mixtures thereof. Other poreforming agents such as manitol, sucrose, lactose, sodium chloride, and pharmaceutical grade excipients also can be included in the fourth layer containing the pH responding polymer, if desired.

The ratio of pH responding polymer to permeability modifying agent in the fourth layer is generally in the range of from 100:0 to 1:100 (w/w).

The following example demonstrate the method manufacturing the compositions described herein and comparative examples of conventional modified release tablets.

### Example 1: Modified release tablet without pH responding polymer.

### [Comparative Example]

Weighed amount of 2-chloro-4-[1-(4-fluoro-phenyl)-2,5-dimethyl-1H-imidazol-4-yl ethynyl]-pyridine and excipients (Pergelatinized starch 1500 for IR formulation; microcrystalline cellulose for Matrix tablet) were mixed in a 1:1 ratio and sieved through 1.0 mm screen. The procedure was repeated three times with portions of excipients, each time at a ration of 1:1. Finally, the rest of excipients were added and blended for another 5 minutes.

An Aeromatic fluid bed granulator MP1^{®} was used for grnaualtion. The described drug and excipients blend from the previous step were filled into the fluid bed granulator. The spray solution consists of Povidone K30^{®} and water.

The following parameters were used:
- Top-spray with a nozzle opening of 1.2 mm
- Inlet air temperature 60-70°C,
- Spraying pressure 2.0-2.2 bar,
- Spraying rate 40 - 45 g/min.

After drying, the granulate was discharged and sieved by FREWITT^{®} Hammer Mill through 1.5mm screen. The milled granules were weighed, and the weight was used to calculate the amount of extra-granular components: talc and magnesium stearate based on the formulation sheet. Talc and magnesium stearate were sieved and manually screened through a 1.0 mm screen and then mixed with a part of the granules (5 times of the amount of talc and magnesium stearate) for 3 min in the tumble mixer. The rest of the granules were added and again mixed for 3 min in the tumble mixer.

The final blend was filled into hard gelatin capsules (size 1) using a ZANASI^{®} 12E filling machine. The final granules were then compressed using a tableting machine and oval shaped tooling and the tablets were coated with using a film-coating machine.

| | Excipients Functionality | IR Capsule Formulation (mg) | Matrix Tablet 1 (mg) | Matrix Tablet 2 (mg) |
|---|---|---|---|---|
| 2-Chloro-4-[1-(4-fluoro-phenyl)-2,5-dimethyl-1H-imidazol-4-ylethynyl]-pyridine | Active Ingredient | 0.6505 | 1.301 | 1.301 |
| Lactose monohydrate | Filler | 109.3 | | 6.7 |
| Lactose spray dried | Filler | - | 71.7 | |
| Microcrystalline cellulose | Matrix former | - | 45.0 | 45.0 |
| Pergelatinized starch1500 | Binder | 60.0 | | |
| HPMC 100000 cp | Rate controlling polymer | - | 15.0 | 60.0 |
| Croscarmellose Na | Disintegrant | 8.0 | | |
| Copovidone VA64^{®} | Binder | - | 11.0 | 11.0 |
| Povidone K30^{®} | Binder | 15.0 | | |
| Mannitol spray dried | Filler | 15.0 | | 70.0 |
| Talc | Glidant | 6.0 | 5.0 | 5.0 |
| Magnesium stearate | Lubricant | 1.0 | 1.0 | 1.0 |
| Total (fill wt) | | 200 | 150 | 200 |

### Example 2: Preparation of modified release tablet containing a pH responding polymer.

2-Chloro-4-[1-(4-fluoro-phenyl)-2,5-dimethyl-1H-imidazol-4-ylethynyl]-pyridine (15.6 g) and lactose monohydrate (878 g) were blended in a Turbula^{®} blender at 40 rpm for 30 minutes. The contents of the blender were passed through a Fitz-mill^{®} Screen #3 with Knife forward speed of ∼2500 rpm. The milled material was transferred to a VG-25 high shear granulator and mixed with Methocel, K100 LV^{®} (600 g), Eudragit L100-55^{®} (720 g), and PVP (120 g), at a speed of 250 rpm (screw) and 1500 rpm (chopper) for two minutes. After two minutes mixing, water was added at a spray rate of 50 g/minute until a consistent granulation was obtained. At the end of granulation, the wet granules were passed through Co-mill^{®} at slow speed of 10 HZ with screen size of Q312R and then transferred to Vector FLM1^{®} fluid bed for drying at 60°C and an air volume of 60 CFM for 2 hours. The dried granules were milled again using Fitz-mill^{®} with 1A screen size and with Knife forward speed of 2500 rpm. The milled granules were weighed and the weight was used to calculate the amount of extragranular components: talc and magnesium stearate. The weighed amount of extragranular excipients was mixed with the milled granules in a Tote^{®} bin blender. The final granules were then compressed using F-press tablet machine and 0.429"x0.1985" oval shaped tooling to give a target hardness of ∼140 N. The tablets were coated with 12% suspension of Opadry^{®} mixture dispersed in purified water using Vector LDCS3^{®} film-coating machine. The resulting tablets have the following composition.

| Ingredients | Excipient Functionality | Amount (mg/tablet) |
|---|---|---|
| 2-Chloro-4-[1-(4-fluoro-phenyl)-2,5-dimethyl-1H-imidazol-4-ylethynyl]-pyridine | Active ingredient | 1.30 |
| Methocel, K100 LV^{®} | Rate controlling polymer | 50.00 |
| Eudragit L100-55^{®} | pH responding polymer | 60.00 |
| Lactose monohydrate | Filler | 73.20 |
| PVP | Binder | 10.00 |
| Talc | Glidant | 4.00 |
| Magnesium stearate | Lubricant | 1.50 |
| Opadry Yellow 03912429^{®} | Top coat | 5.00 |
| Total tablet Weight | | 205.00 |

### Example 3: Preparation of a modified release matrix pellet containing a pH responding polymer, MCC and sodium CMC mixture (F3)

Step 1: A preblended weighed amount of Avicell RC591^{®} (∼173 g) and Eudragit L100-55^{®} (75 g) were blended in a Turbula^{®} blender at 46 rpm for 5 minutes.

Step 2: 2-Chloro-4-[1-(4-fluoro-phenyl)-2,5-dimethyl-1H-imidazol-4-ylethynyl]-pyridine powder (1.6 g) and the polymer blend from Step 1 were mixed in a 1:1 ratio at 46 rpm for 5 minutes. Step 2 was repeated four times with portions of the polymer blend from Step 1. The resulting blend was sieved through 1.0 mm screen, and the screen was rinsed with the remaining polymer blend from Step 1 and blended for another 5 minutes. The blended material was transferred into a Dyazna^{®} vertical high shear granulator. All of the components were mixed for three minutes at a speed of 350 rpm (screw) and 1350 rpm (chopper). After blending for three minutes, the powder mixture was granulated by spraying purified water at 16 g/minute onto the powder mixer in the high shear granulator while continually mixing the contents using an impeller at 350 rpm and the chopper at 1350 rpm until a consistent granulation was obtained. The obtained wet granules were extruded through an LCI Xtruder^{®} Extruder using Screen # 1.0 mm and a speed setting of 40 rpm. The extruded material was transferred into an LUWA^{®} Marumerizer-Spheronizer and spheronized for 5 minutes at 1330 rpm. The spheronized material was collected and dried in a Vector FLM1^{®} fluid bed dryer with an inlet temperature of 60 °C and an air volume of 65 CFM for 1 hours. Using the weight of the obtained pellets, Talc (External component) was weighed and the amount adjusted. The talc was then mixed with the pellets for 5 minutes. The pellets were then filled into #0 opaque white non print gelatin capsules.

| **Compositions (mg)** | **Excipient Functionality** | **Matrix pellets** |
|---|---|---|
| **(1 mg dose)** | | |
| 2-Chloro-4-[1-(4-fluoro-phenyl)-2,5-dimethyl-1H-imidazol-4-ylethynyl]-pyridine | Active ingredient | 1.3 |
| Avicel RC591^{®}(MCC and Sodium CMC blend) | Rate controlling and matrix forming polymer (MCC) and pH responding polymer (Sodium CMC) | 138.7 |
| Eudragit L100-55^{@} | pH responding polymer | 60.0 |
| Talc | Glidant | 3.2 |
| Total fill weight (mg) in a capsule | | 203.2 |

### Example 4: Modified release matrix pellets containing a pH responding polymer and microcrystalline cellulose

| **Compositions (mg)** | **Excipient Functionality** | **Matrix pellets** |
|---|---|---|
| **(1 mg dose)** | | |
| 2-Chloro-4-[1-(4-fluoro-phenyl)-2,5-dimethyl-1H-imidazol-4-ylethynyl]-pyridine | Active ingredient | 1.3 |
| Avicel 101^{®} | Rate controlling and matrix forming polymer | 128.2 |
| Eudragit L100-55^{®} | pH responding polymer | 60.0 |
| Pharmacoat 603^{®} | Binder | 10.0 |
| Talc | Glidant | 0.5 |
| Total fill weight (mg) in a capsule | | 203 |

Weighed the 2-Chloro-4-[1-(4-fluoro-phenyl)-2,5-dimethyl-1H-imidazol-4-ylethynyl]-pyridine powder (7.8 g) and Microcrystalline Cellulose (Avicel, PH-101; 769 g) were weighed, placed into a Turbula^{®} blender, and mixed for 30 minutes at 40 rpm. The contents were passed through a Fitz-mill^{®} Screen #3 with Knife forward speed of ∼2500 rpm. The milled material was transferred to VG-25^{®} high shear granulator and mixed with Eudragit L100-55^{®} (360 g) and Pharmacoat 603^{®} (60 g) at a speed of 250 rpm (screw) and 1500 rpm (chopper) for two minutes. After mixing for two minutes, water was added at a spray rate of 100 g/minutes until a consistent granulation was obtained. The wet granules were extruded through an LCI Xtruder Extruder^{®} using Screen # 1.0 mm and speed setting of 20 rpm. The extruded material was then transferred to a LUWA^{®} Marumerizer-Spheronizer and spheronized for 10 minutes at 1330 rpm. The spheronized material was collected and dried in a fluid bed dryer with an inlet temperature of 60 °C and an air volume of 60 CFM for 3 hours. Using the weight of the obtained pellets, Talc (External component) was weighed and the amount adjusted. The talc was then mixed with the pellets for 5 minutes. The pellets were then filled into #2 opaque white non print gelatin capsules.

### Example 5: Modified release layered pellets (∼5 hr release) with rate controlling and pH responding polymers (F2)

An exemplary bead formulation containing 2-Chloro-4-[1-(4-fluoro-phenyl)-2,5-dimethyl-1H-imidazol-4-ylethynyl]-pyridine as the active ingredient has the following structure:

| **Layer** | **Components** | **Amount** |
|---|---|---|
| Core | Sugar spheres 30/35 | -- |
| Drug layering | Drug suspension with HPMC | 1.2% of core |
| Separation coat | HPMC | 1.5% of core |
| Rate controlling layer | Surelease^{®} (with HPMC as pore former) | 7.7% of core (Surelease/HPMC=7/3) |
| pH controlled layer | Eudragit^{®} L30-D/Talc/TEC | 8.8% of core |
| Top coat | HPMC | 1.7% of core |
| External glidant | Talc | 1.7% of core |

Beads with a multiple-layer coating having the above characteristics were then prepared using the following suspensions. Sugar spheres (500 g) were charged into a Vector FLM1^{®} fluid bed with a Wurster^{®} column and sequentially coated with the amounts of each of the following five coating suspensions in the amounts listed in the table above.

1. A 5 % drug-containing suspension in 5 % hydroxypropylmethyl cellulose (HPMC) solution was applied to the coated beads produced in Step 1 at a nominal product temperature of ∼40-45 °C and 5 minutes of post drying.

The drug layering suspension was prepared in purified water containing the following ingredients:

| | |
|---|---|
| 2-Chloro-4-[1-(4-fluoro-phenyl)-2,5-dimethyl-1H-imidazol-4-ylethynyl]-pyridine | 1.30 mg |
| 10% HPMC Stock Solution | 13.00 mg |
| Purified water | 11.70 mg |

2. A 5 % w/w HPMC separation coat solution was applied at a nominal product temperature of ∼40-45 ° C and 5 minutes of post drying.

The separation coat solution was prepared with the following components:

| | |
|---|---|
| 10% HPMC Stock Solution | 32.60 mg |
| Purified water | 32.60 mg |

3. A Surelease^{®} rate controlling coat dispersion was applied at a nominal product temperature of ∼40-45 ° C and 5 minutes of post drying. After coating, the pellets were cured at 60 °C for 2 hr in forced air oven.

The rate controlling membrane coat dispersion was prepared with the following components:

| | |
|---|---|
| Surelease^{®} Clear, E-7-19040 | 35.44 mg |
| 10% HPMC Stock Solution | 38.00 mg |
| Purified water | 10.96 mg |

4. A Eudragit^{®} L30D-55 pH controlled coat dispersion was applied at nominal product temperature of ∼25-32 °C and 5 minutes of post drying.

The Eudragit^{®} L30D-55 pH controlled coat dispersion was prepared with the following components:

| | |
|---|---|
| Eudragit^{®} L30D-55 | 30.20 mg |
| TEC | 0.91 mg |
| Talc | 4.52 mg |
| Purified water | 36.88 mg |

5. A 5 % w/w HPMC solution was applied at nominal product temperature of ∼35-45 ° C and 5 minutes of post drying. The beads were then cured for 2 hours at 40 ° C in a forced air oven.

6. A top coat solution in purified water was prepared with the following components:

| | |
|---|---|
| 10% HPMC Stock Solution | 18.70 mg |
| Purified water | 18.70 mg |

The resulting beads were fluidized using the following parameters:
Atomization air pressure: 20-40 psi
Partition height: 0.5-1.5 inch
Air volume: 40-60 CFM
Spray rate: 2-15 g/minutes

Using the weight of the coated spheres, the amount of Talc (External component) was weighed and mixed with the coated spheres for 5- minutes. The coated spheres were filled into size #0 hard gelatin capsules to obtain 1 mg of 2-chloro-4-[1-(4-fluoro-phenyl)-2,5-dimethyl-1H-imidazol-4-ylethynyl]-pyridine per capsule.

| Compositions (mg) | 5-Hr drug layered beads with pH controlled layer |
|---|---|
| (1 mg dose) | |
| 2-Chloro-4-[1-(4-fluoro-phenyl)-2,5-dimethyl-1H-imidazol-4-ylethynyl]-pyridine | 1.3 |
| Non-pareil seed | 216.7 |
| Pharmacoat 603^{®} | 13.2 |
| Surelease E-7 19040^{®} | 11.7 |
| Eudragit L30D-55^{®} | 12.0 |
| Tri-ethyl citrate (TEC) | 1.2 |
| Talc | 9.6 |
| Total fill weight (mg) in a capsule | 266 |

### Example 6: Modified release layered pellets with ∼10 hr release with rate controlling and pH responding polymers (F4)

An exemplary bead formulation containing 2-Chloro-4-[1-(4-fluoro-phenyl)-2,5-dimethyl-1H-imidazol-4-ylethynyl]-pyridine as the active ingredient has the following structure:

| **Layer** | **Components** | **Amount** |
|---|---|---|
| Core | Sugar spheres 30/35 | -- |
| Drug layering | Drug suspension with HPMC | 1.6% of core |
| Seal coat | HPMC | 1.5% of core |
| Rate controlling layer | Surelease^{®} (with HPMC as pore former) | 10.3% (Surelease/HPMC=7.5/2 .5) |
| pH controlled layer | Eudragit^{®} L30-D/Talc/TEC | 9% of core |
| Top coat | HPMC | 1.7% of core |
| External glidant | Talc | 1.7% of core |

The layered pellets were prepared in accordance with the method of Example 5.

| **Compositions (mg)** | **10-hr drug layered beads with pH controlled layer** |
|---|---|
| **(1 mg dose)** | |
| 2-Chloro-4-[1-(4-fluoro-phenyl)-2,5-dimethyl-1H-imidazol-4-ylethynyl]-pyridine | 1.3 |
| Non-pareil seed | 162.9 |
| Pharmacoat 603^{®} | 10.7 |
| Surelease E-7 19040^{®} | 12.6 |
| Eudragit L30D-55^{®} | 9.2 |
| Tri-ethyl citrate (TEC) | 0.9 |
| Talc | 7.4 |
| Total fill weight (mg) in a capsule | 205 |

### Example 7: Dug layered beads without pH controlled layer (F1) [Comparative Example]

An exemplary bead formulation containing 2-chloro-4-[1-(4-fluoro-phenyl)-2,5-dimethyl-1H-imidazol-4-ylethynyl]-pyridine as the active ingredient has the following structure:

| **Layer** | **Components** | **Amount** |
|---|---|---|
| Core | Sugar spheres 40/45 | -- |
| Drug layering | Drug suspension with HPMC | 1.6% of core |
| Seal coat | HPMC | 1.5% of core |
| Rate controlling layer | Surelease (with HPMC as pore former and talc) | 31% (Surelease/HPMC/talc=9/1/4.5) |
| pH controlled layer | Eudragit L30-D/talc/TEC | -- |
| Top coat | HPMC | -- |
| External glidant | Talc | 1.4% of core |

The layered pellets were prepared in accordance with the method of Example 5.

| **Compositions (mg)** | **10-hr drug layered beads w/o pH controlled layer** |
|---|---|
| **(1 mg dose)** | |
| 2-Chloro-4-[1-(4-fluoro-phenyl)-2,5-dimethyl-1H-imidazol-4-ylethynyl]-pyridine | 1.3 |
| Non-pareil seed | 162.9 |
| Pharmacoat 603^{®} | 7.1 |
| Surelease E-7 19040^{®} | 30.2 |
| Eudragit L100-55^{®} | - |
| Talc | 19.0 |
| Total fill weight (mg) in a capsule | 220 |

## Claims

1. A pharmaceutical composition comprising a compound of formula I formula I 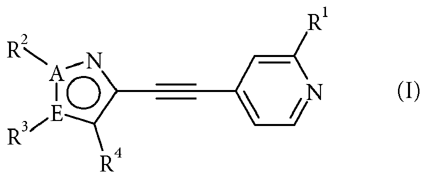 wherein
one of A or E is N and the other is C;
R¹ is halogen or cyano;
R² is lower alkyl;
R³ is aryl or heteroaryl, each of which is optionally substituted by one, two or three substituents chosen from halogen, lower alkyl, lower alkoxy, cycloalkyl, lower haloalkyl, lower haloalkoxy, cyano, or NR'R", or by 1-morpholinyl, 1-pyrrolidinyl, optionally substituted by (CH₂)ₘOR, piperidinyl, optionally substituted by (CH₂)ₘOR, 1,1-dioxo-thiomorpholinyl, piperazinyl, optionally substituted by lower alkyl or (CH₂)ₘ-cycloalkyl;
R is hydrogen, lower alkyl or (CH₂)ₘ-cycloalkyl;
R' and R" are each independently hydrogen, lower alkyl, (CH₂)ₘ-cycloalkyl or (CH₂)ₙOR;
m is 0 or 1;
n is 1 or 2; and
R⁴ is CHF₂, CF₃, C(O)H, or CH₂R⁵, wherein R⁵ is hydrogen, OH, C₁-C₆-alkyl or C₃-C₁₂-cycloalkyl;
and pharmaceutically acceptable salts thereof, a rate-controlling polymer, and a pH responding polymer.

2. A composition according to claim 1, in tablet form.

3. The composition according to any of claims 1 or 2, wherein the particles are further coated with a soluble or insoluble polymer.

4. The composition according to any one of claims 1 to 3, wherein the compound of formula I is present in an amount from about 0.005 % to about 5 % by weight.

5. The composition according to any one of claims 1 to 4, wherein the compound of formula I is present in an amount from about 0.5 % to about 5 %.

6. The composition according to any one of claims 1 to 5, wherein the particle size of the compound of formula 1 is 50 microns or less.

7. The composition according to any one of claims 1 to 6, wherein the particle size of the compound of formula I is 20 microns or less.

8. The composition according to any one of claims 1 to 7, wherein the particle size of the compound of formula I is 10 microns or less.

9. The composition according to any one of claims 1 to 8, wherein the metabotropic glutamate 5 receptor antagonist is a compound of formula Ia 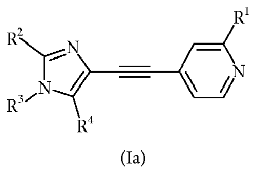 wherein the substituents are described in claim 1.

10. The composition according to any one of claims 1 to 8, wherein the metabotropic glutamate 5 receptor antagonist is a compound of formula Ib 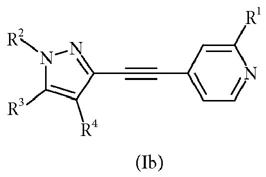 wherein the substituents are described in claim 1.

11. The composition according to any one of claims 1 to 10, wherein the metabotropic glutamate 5 receptor antagonist is a compound selected from the group consisting of
2-[4-(2-Chloro-pyridin-4-ylethynyl)-2,5-dimethyl-1H-imidazol-1-yl]-5-methylpyridine;
2-Chloro-5-[4-(2-Chloro-pyridin-4-ylethynyl)-2,5-dimethyl-1H-imidazol-1-yl]-pyridine;
2-[4-(2-Chloro-pyridin-4-ylethynyl)-2,5-dimethyl-1H-imidazol-1-yl]-6-methyl-4-trifluoromethyl-pyridine;
2-[4-(2-Chloro-pyridin-4-ylethynyl)-2,5-dimethyl-1H-imidazol-1-yl]-pyrazine;
2-[4-(2-Chloro-pyridin-4-ylethynyl)-2,5-dimethyl-1H-imidazol-1-yl]-6-methylpyridine;
2-[4-(2-Chloro-pyridin-4-ylethynyl)-2,5-dimethyl-1H-imidazol-1-yl]-6-(trifluoromethyl)-pyridine;
3-[4-(2-Chloro-pyridin-4-ylethynyl)-2,5-dimethyl-1H-imidazol-1-yl]-5-fluoro-pyridine.
2-Chloro-4-[1-(4-fluoro-phenyl)-2,5-dimethyl-1H-imidazol-4-ylethynyl]-pyridine;
2-Chloro-4-[1-(2,4-difluoro-phenyl)-2,5-dimethyl-1H-imidazol-4-ylethynyl]-pyridine;
2-Chloro-4-[1-(3,5-difluoro-phenyl)-2,5-dimethyl-1H-imidazol-4-ylethynyl]-pyridine;
2-Chloro-4-[1-(4-fluoro-2-methyl-phenyl)-2,5-dimethyl-1H-imidazol-4-ylethynyl]-pyridine;
2-Chloro-4-[1-(4-fluoro-3-methyl-phenyl)-2,5-dimethyl-1H-imidazol-4-ylethynyl]-pyridine;
2-Chloro-4-(2,5-dimethyl-1-p-tolyl-1H-imidazol-4-ylethynyl)-pyridine;
2-Chloro-4-[1-(3-chloro-4-methyl-phenyl)-2,5-dimethyl-1H-imidazol-4-ylethynyl]-pyridine;
2-Chloro-4-[1-(3-fluoro-4-methoxy-phenyl)-2,5-dimethyl-1H-imidazol-4-ylethynyl]-pyridine;
2-Chloro-4-[1-(4-methoxy-phenyl)-2,5-dimethyl-1H-imidazol-4-ylethynyl]-pyridine;
2-Chloro-4-[2,5-dimethyl-1-(4-trifluoromethoxy-phenyl)-1H-imidazol-4-ylethynyl]-pyridine;
2-Chloro-4-[2,5-dimethyl-1-(3-trifluoromethoxy-phenyl)-1H-imidazol-4-ylethynyl]-pyridine;
2-Chloro-4-[2,5-dimethyl-1-(4-trifluoromethyl-phenyl)-1H-imidazol-4-ylethynyl]-pyridine;
2-Chloro-4-[2,5-dimethyl-1-(3-methyl-4-trifluoromethoxy-phenyl)-1H-imidazol-4-ylethynyl]-pyridine;
2-Chloro-4-[1-(4-chloro-phenyl)-2,5-dimethyl-1H-imidazol-4-ylethynyl]-pyridine;
2-Chloro-4-[1-(3-chloro-2-fluoro-phenyl)-2,5-dimethyl-1H-imidazol-4-ylethynyl]-pyridine;
2-Chloro-4-[2,5-dimethyl-1-(3-trifluoromethyl-phenyl)-1H-imidazol-4-ylethynyl]-pyridine;
2-Chloro-4-[1-(3-chloro-4-fluoro-phenyl)-2,5-dimethyl-1H-imidazol-4-ylethynyl]-pyridine;
2-Chloro-4-[2,5-dimethyl-1-(2-methyl-4-trifluoromethoxy-phenyl)-1H-imidazol-4-ylethynyl]-pyridine;
2-Chloro-4-[5-difluoromethyl-1-(4-fluoro-phenyl)-2-methyl-1H-imidazol-4-ylethynyl]-pyridine;
[5-(2-Chloro-pyridin-4-ylethynyl)-3-(4-fluoro-phenyl)-2-methyl-3H-imidazol-4-yl]-methanol;
2-Chloro-4-[1-(4-methoxy-3-trifluoromethyl-phenyl)-2,5-dimethyl-1H-imidazol-4-ylethynyl]-pyridine;
2-Chloro-4-[1-(3,5-difluoro-4-methoxy-phenyl)-2,5-dimethyl-1H-imidazol-4-ylethynyl]-pyridine;
2-Chloro-4-[1-(4-methoxy-3-trifluoromethoxy-phenyl)-2,5-dimethyl-1H-imidazol-4-ylethynyl]-pyridine;
2-Chloro-4-[1-(3-methoxy-4-trifluoromethoxy-phenyl)-2,5-dimethyl-1H-imidazol-4-ylethynyl]-pyridine;
4-{3-[4-(2-Chloro-pyridin-4-ylethynyl)-2,5-dimethyl-imidazol-1-yl]-5-fluoro-phenyl}-morpholine;
2-Chloro-4-[1-(4-fluoro-2-trifluoromethoxy-phenyl)-2,5-dimethyl-1H-imidazol-4-ylethynyl]-pyridine;
2-Chloro-4-[1-(2-fluoro-4-trifluoromethoxy-phenyl)-2,5-dimethyl-1H-imidazol-4-ylethynyl]-pyridine;
2-Chloro-4-[2,5-dimethyl-1-(4-methyl-3-trifluoromethyl-phenyl)-1H-imidazol-4-ylethynyl]-pyridine;
2-Chloro-4-[2,5-dimethyl-1-(3-methyl-4-trifluoromethyl-phenyl)-1H-imidazol-4-ylethynyl]-pyridine;
2-Chloro-4-[2,5-dimethyl-1-(3-methyl-5-trifluoromethyl-phenyl)-1H-imidazol-4-ylethynyl]-pyridine;
2-Chloro-4-[1-(3-methoxy-5-trifluoromethyl-phenyl)-2,5-dimethyl-1H-imidazol-4-ylethynyl]-pyridine;
2-Chloro-4-[1-(3-methoxy-4-trifluoromethyl-phenyl)-2,5-dimethyl-1H-imidazol-4-ylethynyl]-pyridine;
2-Chloro-4-[1-(3,5-dichloro-phenyl)-2,5-dimethyl-1H-imidazol-4-ylethynyl]-pyridine;
2-Chloro-4-[1-(3-chloro-5-methyl-phenyl)-2,5-dimethyl-1H-imidazol-4-ylethynyl]-pyridine;
2-Chloro-4-[1-(3-fluoro-5-methyl-phenyl)-2,5-dimethyl-1H-imidazol-4-ylethynyl]-pyridine;
2-Chloro-4-[1-(3-chloro-5-methoxy-phenyl)-2,5-dimethyl-1H-imidazol-4-ylethynyl]-pyridine;
2-Chloro-4-[1-(3-fluoro-5-methoxy-phenyl)-2,5-dimethyl-1H-imidazol-4-ylethynyl]-pyridine; and
2-Chloro-4-[5-(4-fluoro-phenyl)-1,4-dimethyl-1H-pyrazol-3-ylethynyl]-pyridine.

12. The composition according to any one of claims 1 to 9 and 11, wherein the metabotropic glutamate 5 receptor antagonist is 2-chloro-4-[1-(4-fluoro-phenyl)-2,5-dimethyl-1H-imidazol-4-ylethynyl]-pyridine.

13. The composition of claims 1 to 12, wherein the composition exhibits an in vitro release profile having an NMT of 70 % in one hour, NMT of 85 % in four hours, and of NLT 80 % in 8 hours.

14. The composition according to any of claims 1 to 13, in the form of a matrix tablet which comprises a compound of formula I dispersed in a hydrophilic polymer.

15. The composition according to any one of claims 1 to 14, wherein the hydrophilic polymer is a gel-forming cellulose ether.

16. The composition according to any one of claims 1 to 15, wherein the rate controlling polymer is present in an amount from about 5 % to about 50 % by weight of the composition.

17. The composition according to any one of claims 1 to 16, wherein the rate controlling polymer is present in an amount from about 10 % to about 35 % by weight of the composition.

18. The composition according to any one of claims 1 to 17, wherein the rate controlling polymer is present in an amount from about 10 % to about 25% by weight of the composition.

19. The composition according to any one of claims 1 to 18, wherein the pH responding polymer is present in an amount from about 5 % to about 50 % by weight of the composition.

20. The composition according to any one of claims 1 to 19, wherein the pH responding polymer is present in an amount from about 10 % to about 35 % by weight of the composition.

21. The composition according to any of claims 1 to 20, wherein the pH responding polymer is present in an amount from about 10 % to about 25 % by weight of the composition.

22. The composition according to any of claims 1 to 21, wherein the composition further comprises a filler, surfactant, glidant, lubricant and/or binder.

23. The composition according to any one of claims 1 to 22, wherein the rate controlling polymer is selected from the group consisting of polyvinyl pyrrolidine, hydroxypropyl cellulose, hydroxypropylmethyl cellulose, methyl cellulose, vinyl acetate/crotonic acid copolymers, poly(meth)acrylates, polyvinylacetate, ethyl cellulose, maleic anhydride/methyl vinyl ether copolymers, polyvinylacetate/polidone copolymers and mixtures thereof.

24. The composition according to any one of claims 1 to 23, wherein the pH responding polymer is selected from the group consisting of hydroxyproplymethyl cellulose phthalate, cellulose acetate phthalate, hydroxypropylmethyl cellulose acetate succinate, cellulose acetate trimellitate, ionic poly(meth)acrylates, polyvinyl phthalate, and mixtures thereof.

25. The composition according to any one of claims 1 to 24, which comprises
| **Composition (mg)** | **Amount (mg/tablet)** |
|---|---|
| 2-Chloro-4-[1-(4-fluoro-phenyl)-2,5-dimethyl-1H-imidazol-4-ylethynyl]-pyridine | 1.3 |
| HPMC | 50.0 |
| Poly(meth)acrylate | 50.0 |
| Lactose monohydrate | 83.2 |
| Povidone | 10.0 |
| Talc | 4.0 |
| Magnesium stearate | 1.5 |
| Film-coat | 5.0 |
| Total tablet Weight | 205.0 |

26. The composition according to any one of claims 1 to 13 and 25, wherein the pH responding polymer comprises an ionic polymer.

27. The composition according to any one of claims 1 to 13, 25 and 26, wherein the ionic polymer is poly(meth)acrylate.

28. The composition according to any one of claims 1 to 13 and 25 to 27, wherein the pH responding polymer is present in an amount from about 5 % to about 50 % by weight of the composition.

29. The composition according to any one of claims 1 to 13 and 25 to 28, wherein the pH responding polymer is present in an amount from about 10 % to about 40 % by weight of the composition.

30. The composition according to any one of claims 1 to 13 and 25 to 29, wherein the pH responding polymer is present in an amount from about 25 % to about 35 % by weight of the composition.

31. The composition according to any one of claims 1 to 13 and 25 to 30, which further comprises an insoluble polymer.

32. The composition according to any one of claims 1 to 13 and 25 to 31, wherein the insoluble polymer is selected from ethyl cellulose, polyvinylacetate, or polyvinylacetate/povidone copolymer.

33. The composition according to any one of claims 1 to 13 and 25 to 32, wherein the insoluble polymer is present in an amount from about 5 % to about 50 % by weight of the composition.

34. The composition according to any one of claims 1 to 13 and 25 to 33, wherein the insoluble polymer is present in an amount from about 10 % to about 35 % by weight of the composition.

35. The composition according to any one of claims 1 to 13 and 25 to 34, wherein the insoluble polymer is present in an amount from about 5 % to about 2 5% by weight of the composition.

36. The composition according to any one of claims 1 to 13 and 25 to 35, wherein the composition further comprises a filler, disintegrant, surfactant, glidant, lubricant spheronization enhancer, release modifier and/or binder.
